Europäisches Patentamt

European Patent Office

Office européen des brevets

(19)

(11) Veröffentlichungsnummer: **0 073 054**
**B1**

(12) # EUROPÄISCHE PATENTSCHRIFT

(45) Veröffentlichungstag der Patentschrift:
16.01.85

(51) Int. Cl.⁴: **C 07 C 91/16,** C 07 C 89/04

(21) Anmeldenummer: 82107699.9

(22) Anmeldetag: 23.08.82

(54) Verfahren zur Trennung des Racemats (1RS,2SR)-2-Amino-1-phenyl-propan-1-ol.

(30) Priorität: 28.08.81 DE 3134129

(43) Veröffentlichungstag der Anmeldung:
02.03.83 Patentblatt 83/9

(45) Bekanntmachung des Hinweises auf die Patenterteilung:
16.01.85 Patentblatt 85/3

(84) Benannte Vertragsstaaten:
AT BE CH DE FR GB IT LI NL SE

(56) Entgegenhaltungen:
AT - B - 139 109
CH - A - 565 136
DE - A - 2 120 203
DE - B - 2 258 410
DE - B - 2 558 507

CHEMICAL ABSTRACTS, Band 89, No. 22, 27. November 1978, Columbus, Ohio, USA I.A. YAMSKOV et al. "Ligand-exchange chromatography of racemates, 9. Ligand-Exchange chromatography of amino acid enantiomers on asymmetric sorbents with polydentate sulfur-containing groupings" Seite 21, Abstract No. 180 670n

(73) Patentinhaber: Degussa Aktiengesellschaft,
Weissfrauenstrasse 9, D-6000 Frankfurt am Main 1 (DE)

(72) Erfinder: Bethge, Horst, Schwalbenstrasse 2a,
D-6450 Hanau 1 (DE)
Erfinder: Kleemann, Axel, Dr., Greifenhagenstrasse 9,
D-6450 Hanau 9 (DE)
Erfinder: Martens, Jürgen, Dr., Hochstrasse 5,
D-8755 Alzenau (DE)

## Beschreibung

Die Erfindung betrifft ein Verfahren zur Trennung des Racemats (1RS,2SR)-2-Amino-1-phenylpropan-1-ol, insbesondere zwecks Gewinnung von (1R,2S)-2-Amino-1-phenylpropan-1-ol. Diese Substanz ist ein wichtiges Ausgangsprodukt für die Synthese von Arzneimitteln.

Es ist bekannt, (1R,2S)-2-Amino-1-phenylpropan-1-ol — auch als 1-Norephedrin bezeichnet — mittels optisch aktiver Weinsäure aus dem Racemat (1RS,2SR)-2-Amino-1-phenylpropan-1-ol abzutrennen (Liebigs „Ann. Chem.", 470, 157). Dieses Verfahren ist unbefriedigend, weil die betreffenden diastereomeren Salze in ihrer Löslichkeit wenig verschieden sind.

Es ist ausserdem bekannt, das Racemat (1RS,2SR)-2-Amino-1-phenylpropan-1-ol mittels der optischen Isomeren der 3-Formyl-2,2,5,5-tetramethylthiazolidin-4-carbonsäure zu trennen (DE-PS Nr. 2138121). Die betreffenden diastereomeren Salze weisen zwar verhältnismässig günstige Löslichkeitsunterschiede auf, nachteilig ist bei diesem Verfahren jedoch, dass die Thiazolidincarbonsäure nur wenig beständig ist.

Es ist nun gefunden worden, das Racemat (1RS,2SR)-2-Amino-1-phenylpropan-1-ol mittels der optischen Isomeren des S-(Carboxymethyl)cysteins zu trennen. Das S-(Carboxymethyl)-cystein ist unter den Verfahrensbedingungen sehr beständig, und die diastereomeren Salze unterscheiden sich in ihrer Löslichkeit sehr stark. Die optischen Isomeren des 2-Amino-1-phenylpropan-1-ols fallen bei hohen Ausbeuten in ausgezeichneter optischer und chemischer Reinheit an.

Die optischen Isomeren des S-(Carboxymethyl)-cysteins können aus den entsprechenden optischen Isomeren des Cysteins, beispielsweise durch Umsetzung mit Chloressigsäure in alkalischem Medium nach dem in „J. Org. Chem.", 16 (1951), 749 bis 753, angegebenen Verfahren, hergestellt werden.

Erfindungsgemäss wird das (1R,2S)-2-Amino-1-phenylpropan-1-ol mittels S-(Carboxymethyl)-(R)-cystein und das (1S,2R)-2-Amino-1-phenylpropan-1-ol mittels S-(Carboxymethyl)-(S)-cystein aus dem Racemat abgetrennt. Die sich bildenden Salze aus (1R,2S)-2-Amino-1-phenylpropan-1-ol und S-(Carboxymethyl)-(R)-cystein sowie aus (1S,2R)-2-Amino-1-phenylpropan-1-ol und S-(Carboxymethyl)-(S)-cystein sind bisher nicht beschrieben. Das Salz aus (1R,2S)-2-Amino-1-phenylpropan-1-ol und S-(Carboxymethyl)-(R)-cystein ist erheblich weniger löslich als das dazu diastereomere Salz aus (1S,2R)-2-Amino-1-phenylpropan-1-ol und S-(Carboxymethyl)-(R)-cystein; das Salz aus (1S,2R)-2-Amino-1-phenylpropan-1-ol und S-(Carboxymethyl)-(S)-cystein ist erheblich weniger löslich als das dazu diastereomere Salz aus (1R,2S)-2-Amino-1-phenylpropan-1-ol und S-(Carboxymethyl)-(S)-cystein.

Zur Durchführung des erfindungsgemässen Verfahrens wird in der bei Racemattrennungen üblichen Weise vorgegangen. Das Racemat (1RS, 2SR)-2-Amino-1-phenylpropan-1-ol wird in Gegenwart von Lösungsmitteln mit dem gewünschten optischen Isomeren des S-(Carboxymethyl)-cysteins zusammengebracht, und es werden dann die gebildeten diastereomeren Salze getrennt.

Die zueinander diastereomeren Salze zeigen in zahlreichen Lösungsmitteln ausreichend grosse Löslichkeitsunterschiede. Zu diesen Lösungsmitteln gehört beispielsweise Wasser. Vorzugsweise werden als Lösungsmittel primäre oder sekundäre Alkanole mit bis zu 6 Kohlenstoffatomen oder Äther verwendet und unter diesen Lösungsmitteln insbesondere solche, die mit Wasser unbegrenzt mischbar sind. In Frage kommen beispielsweise Hexan-1-ol, Butan-1-ol, Methyl-tert.-butyläther, insbesondere Methanol, Äthanol, Propan-2-ol, Dioxan und Tetrahydrofuran. Die Lösungsmittel können auch in Gemischen untereinander oder in Gemischen mit Wasser angewendet werden, jedoch werden die Mischungen zweckmässigerweise so gewählt, dass die Lösungsmittel nicht mehr als eine Phase bilden.

Das Racemat (1RS,2SR)-2-Amino-1-phenylpropan-1-ol wie auch das betreffende optische Isomere des S-(Carboxymethyl)cysteins können in fester Form oder als Aufschlämmung oder Lösung in dem Lösungsmittel eingesetzt werden. Das optische Isomere des S-(Carboxymethyl)cysteins und das Racemat (1RS,2SR)-2-Amino-1-phenylpropan-1-ol können zwar in beliebigem Mengenverhältnis zueinander eingesetzt werden, es ist im allgemeinen jedoch zweckmässig, je Mol des Racemats nicht weniger als etwa 0,5 und nicht mehr als etwa 5,0 mol des optischen Isomeren zu nehmen. Vorzugsweise werden je Mol des Racemats 0,9 bis 1,1, insbesondere 1,0 mol des optischen Isomeren angewendet. Es kann bei allen Temperaturen gearbeitet werden, bei denen die Lösungsmittel in flüssiger Form vorliegen.

Zur Trennung der diastereomeren Salze wird vorzugsweise in der bei einer fraktionierten Kristallisation üblichen Weise vorgegangen. Es wird die Mischung auf erhöhte Temperaturen, vorzugsweise auf Temperaturen nahe dem Siedepunkt, gebracht, so viel Lösungsmittel angewendet, dass alle Substanzen gelöst sind, und schliesslich die Lösung zur Kristallisation abgekühlt.

Aus den anfallenden Salzen aus (1R,2S)-2-Amino-1-phenylpropan-1-ol und S-(Carboxymethyl)-(R)-cystein oder (1S,2R)-2-Amino-1-phenylpropan-1-ol und S-(Carboxymethyl)-(S)-cystein wird das betreffende 2-Amino-1-phenylpropan-1-ol freigesetzt, indem die Salze mit starker Säure, vorzugsweise starker Mineralsäure, wie Chlorwasserstoffsäure, behandelt werden.

*Beispiele:*

Die gewonnenen optisch aktiven Substanzen wurden jeweils auf ihren spezifischen Drehwert $[\alpha]_D^T$ untersucht. Dieser wird in Grad·cm³/dm·g angegeben. Prozentangaben bedeuten Gewichtsprozente.

1. Eine Mischung von 50 g (0,28 mol) S-(Carboxymethyl)-(R)-cystein und 42,5 g (0,28 mol) (1RS,2SR)-2-Amino-1-phenylpropan-1-ol wurde in 50 ml Wasser gelöst. Die Lösung wurde auf 30 bis 40°C gehalten und tropfenweise mit Äthanol versetzt, bis Kristallisation einsetzte, dann langsam auf 5°C abgekühlt und unter Absaugen filtriert. Der Rückstand wurde mit 150 ml Äthanol, das 10% Wasser enthielt, gewaschen und bei 50°C und 25 mbar getrocknet. Die gewonnene Substanz war das Salz aus S-(Carboxymethyl)-(R)-cystein und (1R,2S)-2-Amino-1-phenylpropan-1-ol- Die Ausbeute betrug 44,8 g, entsprechend 97%, bezogen auf das in dem eingesetzten Racemat enthaltene (1R,2S)-2-Amino-1-phenylpropan-1-ol. Der Schmelzpunkt des Salzes war 66°C und der spezifische Drehwert −37,2° (T = 20°C; c = 1 in Wasser).

Das Salz wurde in 150 ml Wasser gelöst, und die Lösung wurde bei 25°C mittels 2N-Schwefelsäure auf den pH-Wert 2,8 eingestellt. Hierbei wurde das S-(Carboxymethyl)-(R)-cystein ausgefällt. Es wurde abfiltriert. Das Filtrat wurde mit 200 ml einer 50%igen wässerigen Natriumhydroxidlösung versetzt. Die Mischung wurde dreimal mit je 100 ml Methylenchlorid extrahiert. Die vereinigten Extrakte wurden mit Natriumsulfat getrocknet. Dann wurde das Methylenchlorid abgetrieben und der Rückstand in Methyl-tert.-butyläther umkristallisiert. Gewonnen wurden 19,6 g (1R,2S)-2-Amino-1-phenylpropan-1-ol, entsprechend 95% Ausbeute, bezogen auf das in dem eingesetzten Racemat enthaltene (1R,2S)-2-Amino-1-phenyl-propan-1-ol. Der spezifische Drehwert der gewonnenen Substanz war −13,9° (T = 20°C; c = 1 in wasserfreiem Äthanol).

Das beim Abfiltrieren des Salzes aus S-(Carboxymethyl)- und (1R,2S)-2-Amino-1-phenylpropan-1-ol verbliebene Filtrat wurde mit 2N wässeriger Chlorwasserstoffsäure auf den pH-Wert 3,0 eingestellt. Hierbei wurde S-(Carboxymethyl)-(R)-cystein abgeschieden. Die Substanz wurde abfiltriert, mit dem zuvor aus dem Salz freigesetzten S-(Carboxymethyl)-(R)-cystein vereinigt, dann mit 50 ml 0,001 N wässeriger Chlorwasserstoffsäure gewaschen und bei 105°C getrocknet. Es wurden 47,5 g, entsprechend 95%, des eingesetzten S-(Carboxymethyl)(R)-cysteins zurückgewonnen. Die Substanz konnte unmittelbar für weitere Racemattrennungen verwendet werden.

Das zuletzt bei dem Abfiltrieren des S-(Carboxymethyl)-(R)-cysteins verbliebene Filtrat wurde mit 200 ml 50%iger wässeriger Natriumhydroxidlösung versetzt. Die Mischung wurde dreimal mit je 100 ml Methylenchlorid extrahiert. Die vereinigten Extrakte wurden mit Natriumsulfat getrocknet. Dann wurde das Methylenchlorid abgetrieben und der Rückstand in Diisopropyläther umkristallisiert. Gewonnen wurden hierbei 14,5 g (1S,2R)-2-Amino-1-phenylpropan-1-ol, entsprechend 71% Ausbeute, bezogen auf das mit dem Racemat eingesetzte (1S,2R)-2-Amino-1-phenylpropan-1-ol. Der spezifische Drehwert der gewonnenen Substanz war +14,4° (T = 27°C; c = 4 in wasserfreiem Äthanol).

2. 50 g (0,28 mol) S-(Carboxymethyl)-(R)-cystein wurden in 400 ml Methanol suspendiert, das 10% Wasser enthielt. Dieser Suspension wurde 42,5 g (0,28 mol) (1RS,2SR)-2-Amino-1-phenylpropan-1-ol zugesetzt. Die Mischung wurde währenddessen auf 40 bis 50°C und danach 1 h lang unter Rückfluss auf Siedetemperatur gehalten, dann im Verlauf von 30 min auf 23°C abgekühlt und unter Absaugen filtriert. Der Rückstand wurde mit 200 ml Methanol gewaschen und bei 50°C und 25 mbar getrocknet. Gewonnen wurden 41,6 g des Salzes aus S-(Carboxymethyl)-(R)-cystein und (1R,2S)-2-Amino-1-phenylpropan-1-ol, entsprechend 90% Ausbeute, bezogen auf das mit dem Racemat eingesetzte (1R,2S)-2-Amino-1-phenylpropan-1-ol. Der Schmelzpunkt war 68°C und der spezifische Drehwert −38,2° (T = 20°C; c = 1 in Wasser).

Das Salz wurde bei 25°C in 150 ml Wasser gelöst, und die Lösung wurde mittels 2N wässeriger Chlorwasserstoffsäure auf den pH-Wert 3,0 eingestellt. Hierbei wurde das S-(Carboxymethyl)-(R)-cystein ausgefällt. Es wurde unter Absaugen filtriert. Gewonnen wurden 20,3 g, entsprechend 90%, bezogen auf das eingesetzte S-(Carboxymethyl)-(R)-cystein. Das Filtrat wurde zur Trockne eingedampft. Es fielen 21,9 g (1R,2S)-2-Amino-1-phenylpropan-1-olhydrochlorid an, entsprechend 98% Ausbeute, bezogen auf das mit dem Racemat eingesetzte (1R,2S)-2-Amino-1-phenylpropan-1-ol. Die gewonnene Substanz wurde in Propan-2-ol umkristallisiert. Dann war der Schmelzpunkt 165°C und der spezifische Drehwert −36° (T = 20°C; c = 1 in Wasser).

3. Es wurde wie nach Beispiel 2 verfahren, jedoch wurde das S-(Carboxymethyl)-(R)-cystein in 1000 ml wasserfreiem Methanol eingesetzt. Es wurden 37,0 g des Salzes aus (S-Carboxymethyl)-(R)-cystein und (1R,2S)-2-Amino-1-phenylpropan-1-ol gewonnen, entsprechend 80% Ausbeute. Der Schmelzpunkt war 67°C und der Drehwert −37,5° (T = 20°C; c = 1 in Wasser).

*Elementaranalyse für* $C_{14}H_{22}N_2O_5S$:
Ber.:      C 50,89    H 6,71    N 8,48    S 9,71%
Gef.:      C 50,65    H 6,70    N 8,50    S 9,79%

4. Es wurde wie nach Beispiel 1 verfahren, jedoch die Mischung der Ausgangssubstanzen in 800 ml Dioxan suspendiert, die Suspension unter Rückfluss auf Siedetemperatur gehalten und hierbei mit so viel Wasser versetzt, dass alle Substanzen vollständig gelöst wurden. Die Lösung wurde dann im Verlauf von 2 h auf 18°C gekühlt. Gewonnen wurden 36,1 g des Salzes aus S-(Carboxymethyl)-(R)-cystein und (1R,2S)-2-Amino-1-phenylpropan-1-ol, entsprechend 78% Ausbeute. Der Schmelzpunkt war 68°C und der Drehwert −37,4° (T = 20°C; c = 1 in Wasser).

5. Es wurde wie nach Beispiel 4 verfahren, jedoch wurden statt Dioxan 800 ml Propan-2-ol als Lösungsmittel angewendet. Gewonnen wurden 43,9 g des Salzes aus S-(Carboxymethyl)-(R)-cystein und (1R,2S)-2-Amino-1-phenylpropan-1-ol, entsprechend 95% Ausbeute. Der Schmelzpunkt war 68°C und der Drehwert −38,1° (T = 20°C, c = 1 in Wasser).

6. Es wurde wie nach Beispiel 4 verfahren, jedoch wurden statt Dioxan 200 ml Tetrahydrofuran als Lösungsmittel angewendet. Gewonnen wurden 44,4 g des Salzes aus S-(Carboxymethyl)-(R)-cystein und (1R,2S)-2-Amino-1-phenylpropan-1-ol, entsprechend 96% Ausbeute. Der Schmelzpunkt war 60°C und der Drehwert −38,8° (T = 20°C; c = 1 in Wasser).

7. Es wurde wie nach Beispiel 1 verfahren, jedoch wurde ein Gemisch aus 100 g (0,56 mol) S-(Carboxymethyl)-(R)-cystein und 124 g (0,82 mol) (1RS,2SR)-2-Amino-1-phenylpropan-1-ol in 800 ml Methanol eingesetzt, das 2% Wasser enthielt. Die Mischung wurde 1 h lang unter Rückfluss auf Siedetemperatur gehalten und dann im Verlaufe von 30 min auf 30°C abgekühlt. Gewonnen wurden 89,6 g, entsprechend 97%, des Salzes aus S-(Carboxymethyl)-(R)-cystein und (1R,2S)-2-Amino-1-phenylpropan-1-ol. Der Schmelzpunkt des Salzes war 67°C und der Drehwert −37,3° (T = 20°C; c = 1 in Wasser).

8. Es wurde wie nach Beispiel 1 verfahren, jedoch wurden statt S-(Carboxymethyl)-(R)-cystein 50,0 g (0,28 mol) S-(Carboxymethyl)-(S)-cystein eingesetzt. Gewonnen wurde das Salz aus S-(Carboxymethyl)-(S)-cystein und (1S,2R)-2-Amino-1-phenylpropan-1-ol. Die Ausbeute betrug 44,0 g, entsprechend 95%, bezogen auf das in dem eingesetzten Racemat enthaltene (1S,2R)-2-Amino-1-phenylpropan-1-ol. Der spezifische Drehwert des gewonnenen Salzes war +37,3° (T = 20°C, c = 1 in Wasser).

*Elementaranalyse für* $C_{14}H_{22}N_2O_5S$:

| | | | | | |
|---|---|---|---|---|---|
| Ber.: | C 50,89 | H 6,71 | N 8,48 | S 9,71% |
| Gef.: | C 50,70 | H 6,65 | N 8,59 | S 9,59% |

Das Salz wurde in 100 ml Wasser gelöst, und die Lösung wurde mittels 2N wässeriger Chlorwasserstoffsäure auf den pH-Wert 3,0 eingestellt. Hierbei wurde das S-(Carboxymethyl)-(S)-cystein ausgefällt. Es wurde abfiltriert. Das Filtrat wurde zur Trockne eingedampft. Der Rückstand wurde in Propan-2-ol umkristallisiert. Gewonnen wurden 24,0 g (1S,2R)-2-Amino-1-phenylpropan-1-olhydrochlorid, entsprechend 96% Ausbeute, bezogen auf das mit dem Racemat eingesetzte (1S,2R)-2-Amino-1-phenylpropan-1-ol. Der Schmelzpunkt war 174 bis 176°C und der spezifische Drehwert +33,4° (T = 23°C; c = 7 in Wasser).

Das beim Abfiltrieren des Salzes aus S-(Carboxymethyl)-(S)-cystein und (1S,2R)-2-Amino-1-phenylpropan-1-ol verbliebene Filtrat wurde zur Trockne eingedampft. Hierbei fielen 44 g des Salzes aus S-(Carboxymethyl)-(S)-cystein und (1R,2S)-2-Amino-1-phenylpropan-1-ol an, entsprechend 95% Ausbeute, bezogen auf das mit dem Racemat eingesetzte (1R,2S)-2-Amino-1-phenylpropan-1-ol.

Das Salz wurde in 100 ml Wasser gelöst, und die Lösung wurde mittels 2N wässeriger Chlorwasserstoffsäure auf den pH-Wert 3,0 eingestellt. Hierbei wurde das S-(Carboxymethyl)-(S)-cystein ausgefällt. Es wurde abfiltriert. Das Filtrat wurde zur Trockne eingedampft. Der Rückstand wurde in Propan-2-ol umkristallisiert. Gewonnen wurden 18,7 g (1R,2S)-2-Amino-1-phenylpropan-1-olhydrochlorid, entsprechend 71% Ausbeute, bezogen auf das mit dem Racemat eingesetzte (1R,2S)-2-Amino-1-phenylpropan-1-ol. Der spezifische Drehwert war −31,1° (T = 20°C; c = 1 in Wasser).

## Patentansprüche

1. Trennung des Racemats (1RS,2SR)-2-Amino-1-phenylpropan-1-ol mittels der optischen Isomeren des S-(Carboxymethyl)cysteins.

2. Salz aus (1R,2S)-2-Amino-1-phenylpropan-1-ol und S-(Carboxymethyl)-(R)-cystein.

3. Salz aus (1S,2R)-2-Amino-1-phenylpropan-1-ol und S-(Carboxymethyl)-(S)-cystein.

## Claims

1. The resolution of the racemate (1RS,2RS)-2-amino-1-phenyl-propan-1-ol using the optical isomers of the S-(carboxymethyl)-cysteine.

2. Salt of (1R,2S)-2-amino-1-phenyl-propan-1-ol and S-(carboxymethyl)-(R)-cysteine.

3. Salt of (1S,2R)-2-amino-1-phenyl-propan-1-ol and S-(carboxymethyl)-(S)-cysteine.

## Revendications

1. Dédoublement du racémate (1RS,2SR)-2-amino-1-phénylpropan-1-ol au moyen des isomères optiques de la S-(carboxyméthyl)cystéine.

2. Sel de (1R,2S)-2-amino-1-phénylpropan-1-ol et de S-(carboxyméthyl)-(R)-cystéine.

3. Sel de (1S,2R)-2-amino-1-phénylpropan-1-ol et de S-(carboxyméthyl)-(S)-cystéine.